# EUROPÄISCHE PATENTSCHRIFT

(11) **EP 2 026 865 B1**
(45) Veröffentlichungstag und Bekanntmachung des Hinweises auf die Patenterteilung: **30.03.2016**
(21) Anmeldenummer: 07701874.5
(22) Anmeldetag: 13.02.2007
(51) Int. Cl.: A61M 25/02, A61M 25/06, A61M 5/158, A61M 39/10

(54) **ANORDNUNG ZUM ZUFÜHREN EINER FLÜSSIGKEIT IN DEN KÖRPER EINES PATIENTEN**
ARRANGEMENT FOR INTRODUCING A LIQUID INTO THE BODY OF A PATIENT
SYSTÈME D'INTRODUCTION DE LIQUIDE DANS L'ORGANISME D'UN PATIENT

(30) Priorität: 07.06.2006 CH 911062006
(43) Veröffentlichungstag der Anmeldung: 25.02.2009
(73) Patentinhaber: F. Hoffmann-La Roche AG, 4070 Basel (CH); Roche Diagnostics GmbH, 68305 Mannheim (DE)
(72) Erfinder: WYSS, Martin, CH-3510 Konolfingen (CH); SCHEURER, Simon, 3006 Bern (CH); AESCHLIMANN, Reto, 3426 Aefligen (CH); THALMANN, Christian, 6365 Kehrsiten (CH)
(74) Vertreter: Rentsch Partner AG
(86) Internationale Anmeldenummer: PCT/CH2007/000074
(87) Internationale Veröffentlichungsnummer: WO 2007/140631

(56) Entgegenhaltungen:
- EP-A1- 1 820 525
- WO-A-98/58693
- WO-A-02/070037
- WO-A-2004/101071
- US-A1- 2007 185 441

## Beschreibung

### TECHNISCHES GEBIET

Die vorliegende Erfindung betrifft eine Anordnung zum Zuführen einer Flüssigkeit in den Körper eines Patienten oder zur Abführung einer Flüssigkeit aus dem Körper eines Patienten gemäss den Oberbegriffen des unabhängigen Patentanspruchs.

### STAND DER TECHNIK

Bei Patienten, welche einen kontinuierlichen Bedarf an durch direkte Zuführung in das Körpergewebe oder in den Blutkreislauf verabreichbaren Medikamenten haben oder bei denen über einen längeren Zeitraum Körperflüssigkeitsproben zur Überwachung bestimmter Parameter, wie z.B. des Blutzuckers, aus dem Körper entnommen werden müssen, ist es sinnvoll, die Zuführung des Medikaments oder die Entnahme der Körperflüssigkeit über eine an geeigneter Stelle in den Körper eingebrachte und über einen längeren Zeitraum dort verbleibende Kanüle vorzunehmen.

So erfolgt heute bei vielen Patienten mit Diabetes Typ I und Typ II die Zuführung der erforderlichen Insulinmenge zum Körper kontinuierlich oder in kleinen Zeitabständen, indem eine durch die Haut in das subkutane Gewebe des Patienten eindringende Kanüle, welche Teil einer auf dem Körper des Patienten befestigten und als "Infusionsset" bezeichneten Anordnung ist, durch eine automatisierte Insulinpumpe kontrolliert mit Insulin versorgt wird. Die Verbindung zwischen Insulinpumpe und Infusionsset wird dabei über eine flexible Zuführungsleitung hergestellt, welche mittels eines Konnektors lösbar am Infusionsset angekoppelt wird. Da das Infusionsset praktisch permanent am Körper getragen wird, ist es bei solchen Anwendungen wichtig, dass dieses einen guten Tragekomfort bietet. Hierzu ist es insbesondere wichtig, dass das Infusionsset mit konnektierter Zuführungsleitung möglichst flach baut und die Bewegungsfreiheit des Patienten möglichst wenig beeinträchtigt. Zudem ist es wichtig, dass das An- und Abkoppeln der Zuführungsleitung auf einfache und sichere Weise möglich ist und Bedienungsfehler möglichst schon durch die konstruktive Ausgestaltung des Infusionssets ausgeschlossen werden. Ähnliche Rahmenbedingungen und Anforderungen ergeben sich, wenn über einen längeren Zeitraum Körperflüssigkeitsproben aus dem Körper eines Patienten entnommen werden sollen.

Die Dokumente WO 02/070037 A2, DE 299 05 068 U1, US 6,923,791 B2, WO 2005/049117 A2, US 2005/0101910 A1 und WO 2004/026375 A1 offenbaren Infusionsvorrichtungen,. bei denen die Zuführungsleitung mit einem Adapterteil in axialer Richtung der Kanüle auf den die Kanüle tragenden Teil der Vorrichtung aufgesteckt und in entgegengesetzter Richtung wieder von diesem entfernt werden kann. Dabei ist die Kanüle bei den in WO 02/070037 A2, US 6,923,791 B2, WO 2005/049117 A2, US 2005/0101910 A1 und WO 2004/026375 A1 offenbarten Infusionsvorrichtungen zum im wesentlichen senkrechten Eindringen in den Körper des Patienten vorgesehen, wobei die Zuführungsleitung in einer Richtung senkrecht zur Aufsteckrichtung vom Adapterteil weggeführt wird, und bei der in DE 299 05 068 U1 offenbarten Vorrichtung zum Eindringen in den Körper eines Patienten unter einem flachen Winkel vorgesehen, wobei die Zuführungsleitung in Aufsteckrichtung vom Adapterteil weggeführt wird. Nach dem Anstecken an das die Kanüle tragende Teil ist der Adapterteil bei den Vorrichtungen gemäss WO 02/070037 A2, DE 299 05 068 U1, US 6,923,791 B2, WO 2005/049117 A2, US 2005/0101910 A1 drehbar gegenüber dem die Kanüle tragenden Teil, während er bei der in WO 2004/026375 A1 offenbarten Vorrichtung in der beim Aufstecken gewählten rotatorischen Ausrichtung bezüglich des die Kanüle tragenden Teils arretiert ist.

Alle diese bekannten Vorrichtungen weisen den Nachteil auf, dass der Adapter zum Abkoppeln der Zuführungsleitung vom Infusionsset in axialer Richtung der Kanüle vom Infusionsset entfernt wird, wodurch eine nicht unerhebliche Gefahr besteht, dass die Kanüle unbemerkt ganz oder teilweise aus der Einstichstelle herausgezogen wird und anschliessend eine Zuführung von flüssigem Medikament in das Gewebe nicht oder nur unzureichend möglich ist.

Die in WO 02/070037 A2, US 6,923,791 B2, WO 2005/049117 A2, US 2005/0101910 A1 und WO 2004/026375 A1 offenbarten Infusionsvorrichtungen weisen weiter den Nachteil auf, dass sie im applizierten Zustand bei angekoppelter Zuführungsleitung relativ hoch bauen und die in DE 299 05 068 U1 offenbarte Vorrichtung weist zudem den Nachteil auf, dass nach der Applikation des die Kanüle tragenden Teils am Körper eines Patienten keine Möglichkeit mehr besteht, die Richtung, in welcher die Zuführungsleitung von der Infusionsvorrichtung wegführt, zu verändern.

Eine weiter Infusionsvorrichtung wird in der WO 2004/10107 A1 offenbart, welche zweiteilig ausgeführt ist und eine auf der Hautoberfläche des Patienten zu platzierenden Basis sowie ein Anschlussteil umfasst, welches mit der Basis fluidisch gekoppelt wird. Die Kopplung erfolgt dabei axial zur von der Basis abstehenden Kanüle, wobei Basis und Anschlussteil frei gegeneinander um die Kanülenachse drehbar sowie abkoppelbar und wieder verbindbar sind. Eine Leitung zur Zuführung einer Flüssigkeit ist offenbarungsgemäss fest mit dem Anschlussteil verbunden. Eine ähnliche Vorrichtung wird in der WO 2007/092210 A1 offenbart, welche bezüglich der vorliegenden Anmeldung Stand der Technik gemäss Art. 54(3, 4) EPÜ 1973 darstellt.

Die EP 1820525 A1, welche bezüglich der vorliegenden Anmeldung ebenfalls Stand der Technik gemäss Art. 54(3, 4) EPÜ 1973 darstellt, offenbart eine Infusionsvorrichtung, bei welcher eine flexible Kanüle zunächst senkrecht in die Haut des Patienten eingestochen und der oberhalb der Hautoberfläche befindliche Teil der flexiblen Kanüle anschliessend mittels eines Gelenks um eine senkrecht zur Kanüle stehende Achse in eine Orientierung parallel zur Hautoberfläche umgelenkt bzw. gekippt und verrastet wird, worauf anschliessend mittels einer Kupplung eine Leitung zur Zuführung einer Flüssigkeit angekoppelt wird.

### DARSTELLUNG DER ERFINDUNG

Es stellt sich daher die Aufgabe, eine Anordnung zum Zuführen einer Flüssigkeit in den Körper eines Patienten oder zum Entnehmen einer Flüssigkeit aus dem Körper eines Patienten zur Verfügung zu stellen, welche die Nachteile des Standes der Technik nicht aufweist oder diese zumindest teilweise vermeidet.

Diese Aufgabe wird durch die Anordnung gemäss Patentanspruch 1 gelöst.

Demgemäss umfasst die erfindungsgemässe Anordnung zum Zuführen einer Flüssigkeit in den Körper eines Patienten bzw. zum Abführen einer Flüssigkeit aus dem Körper eines Patienten ein erstes Bauteil, welches eine von diesem abstehende Kanüle trägt, die zur Anordnung im Körper eines Patienten nach einem Einstechen derselben durch die Haut vorgesehen ist. Weiter umfasst die Anordnung ein zweites Bauteil, welches einen Anschlussport zum lösbaren Ankoppeln eines entsprechenden Konnektors einer Leitung zum Zu- oder Abführen einer Flüssigkeit bildet. Unter einem "entsprechenden" Konnektor wird hier ein Anschlusselement verstanden, welches auf den Anschlussport angepasst ist, derartig, dass der Anschlussport und der Konnektor zusammen eine sichere und mehrfach wieder lösbare und wieder konnektierbare Ankopplungsstelle für die Flüssigkeitsleitung in der Art einer aufeinander abgestimmten Stecker- und Buchseverbindung ergeben. Das erste Bauteil und das zweite Bauteil der erfindungsgemässen Anordnung sind derartig ausgestaltet und miteinander verbunden, dass sie untrennbar sind und um eine Drehachse herum relativ zueinander verdrehbar sind. Dabei bilden das erste und das zweite Bauteil in jeder Relativposition, die sie zueinander einnehmen können, einen gegen aussen abgedichteten Kanal zwischen dem Anschlussport und der Kanüle, über welchen eine Flüssigkeit vom Anschlussport zur Kanüle oder von der Kanüle zum Anschlussport geführt werden kann. Der Anschlussport ist dabei derartig ausgebildet und am zweiten Bauteil angeordnet, dass das An- und Abkoppeln eines entsprechenden Konnektors einer Leitung ausschliesslich in einer Richtung quer zur Längsachse der Kanüle möglich ist.

Durch die Erfindung wird die Bereitstellung von Anordnungen zum Zuführen einer Flüssigkeit in den Körper eines Patienten bzw. zum Entnehmen einer Flüssigkeit aus dem Körper eines Patienten möglich, welche einen hohen Tragekomfort bei gleichzeitig guter Sicherheit gegen Bedienungsfehler bieten.

In einer bevorzugten Ausführungsform der Anordnung ist der Anschlusssport des zweiten Bauteils der Anordnung derartig ausgebildet, dass das Ankoppeln und Abkoppeln eines entsprechenden Konnektors jeweils senkrecht zur Längsachse der Kanüle erfolgen muss. Hierdurch lässt sich eine maximale Sicherheit gegen ein unbeabsichtigtes Herausziehen der Kanüle beim Abkoppeln des Konnektors erreichen.

Erfindungsgemäss sind das erste und das zweite Bauteil um eine parallel zur Längsachse der Kanüle verlaufende Rotationsachse relativ zueinander verdrehbar. Auf diese Weise lassen sich besonders einfache Konstruktionen realisieren, insbesondere dann, wenn die Kanüle zum senkrechten Eindringen in einen Körper vorgesehen ist, was bevorzugt ist.

In noch einer weiteren bevorzugten Ausführungsform der Anordnung steht die Kanüle senkrecht oder nicht-senkrecht von einer im wesentlichen ebenen Aussenseite des ersten Bauteils ab, wobei in beiden Fällen das erste und das zweite Bauteil um eine senkrecht zu dieser Aussenseite stehende Rotationsachse relativ zueinander verdrehbar sind. Hierdurch ergibt sich der Vorteil, dass sich die Rotationsebene des zweiten Bauteils nach erfolgtter Applikation der Anordnung parallel zur Körperoberfläche erstreckt, so dass sich bei jeder rotatorischen Relativposition zwischen dem ersten und dem zweiten Bauteil eine im wesentlichen identische Applilcationssituation ergibt.

In noch einer weiteren bevorzugten Ausführungsform der Anordnung sind das erste und das zweite Bauteil um 360° verdrehbar, bevorzugterweise endlos umeinander rotierbar, oder um weniger als 360° zueinander verdrehbar, d.h. nicht endlos umeinander rotierbar. Je nach Anwendung kann die eine oder die andere Variante vorteilhaft sein, wobei erstgenannte Variante den Vorteil birgt, dass das zweite Bauteil in jeder Position angeordnet werden kann und dass bei endlos umeinander rotierbaren ersten und zweiten Bauteilen und entsprechender Ausgestaltung der Anordnung die Rotation des zweiten Bauteils in eine beliebige andere Relativposition in beliebiger Rotationsrichtung erfolgen kann. Letztgenannte Variante weist beispielsweise den Vorteil auf, dass ein unerwünschtes "Aufwickeln" der bei bestimmungsgemässer Verwendung mit der Anordnung verbundenen Leitung infolge einer mehrfachen Rotation des zweiten Bauteils relativ zum ersten Bauteil nicht möglich ist.

In noch einer weiteren bevorzugten Ausführungsform der Anordnung ist die Verbindung zwischen dem ersten und dem zweiten Bauteil derartig ausgebildet, dass zum Gegeneinanderverdrehen der beiden Bauteile relativ zu einander ein bestimmtes Drehmoment überwunden werden muss, bevorzugterweise ein Drehmoment im Bereich zwischen 0,01 und 0,05 Nm, noch bevorzugter im Bereich zwischen 0,01 und 0,1 Nm.

Dabei ist es bevorzugt, dass zur Erzeugung dieses zu überwindenden Drehmoments eine Anordnung mit einem vorgespannten elastischen Bauteil vorhanden ist, welche Reibung zwischen dem ersten und dem zweiten Bauteil erzeugt, und zwar bevorzugterweise eine vorgespannte elastische Abdichtung, wie z.B. eine O-Ring-Abdichtung, und/oder eine Ratschenanordnung.

Solche Ausführungsformen weisen den Vorteil auf, dass ein unnötiges, z.B. durch Lageänderung der die Anordnung tragenden Person bezüglich der Schwerkraftrichtung bewirktes Verdrehen der beiden Bauteile zueinander verhindert werden kann, wodurch sich bei bestimmten Ausführungsformen der Anordnung die bereits zuvor erwähnte Gefahr eines "Aufwickelns" einer an die Anordnung angekoppelten Flüssigkeitsleitung deutlich reduzieren lässt. Auch lässt sich hierdurch die Wiederauffindbarkeit des Anschlussports nach einem vorübergehenden Abkoppeln des Konnektors der Anschlussleitung von der Anordnung verbessern, da das zweite Bauteil in Abwesenheit von an diesem angreifenden Kräften in der bei der Abkopplung eingenommenen Relativposition gegenüber dem ersten Bauteil verbleibt.

Erfindungsgemäss weist die Anordnung Arretierungsmittel auf, welche ausgestaltet sind zur Aufhebung der Verdrehbarkeit des ersten und zweiten Bauteils relativ zueinander durch Arretieren derselben in einer bestimmten Relativposition zueinander.

Dabei ist es gemäss einer ersten Variante bevorzugt, wenn die Arretierungsmittel derartig ausgestaltet sind, dass das erste und das zweite Bauteil automatisch in einer Relativposition zueinander arretiert sind, wenn kein entsprechender Konnektor einer Leitung an den Anschlussport des zweiten Bauteils angekoppelt ist bzw. dass beide Bauteile beim Abkoppeln des Konnektors in der gerade zueinander eingenommenen Relativposition arretiert werden, und dass die Arretierung durch Ankoppeln eines entsprechenden Konnektors an den Anschlussport aufhebbar ist, z.B. indem ein Arretierungselement der Arretierungsvorrichtung durch den Konnektor in eine Nichtarretierungsposition bewegt wird. Eine solche Ausgestaltung garantiert die einfache Wiederauffindbarkeit des Anschlussports nach einem vorübergehenden Abkoppeln des Konnektors von der Anordnung, da das zweite Bauteil in seiner bei der Abkopplung eingenommenen Relativposition verbleibt.

Gemäss einer zweiten Variante ist es bevorzugt, wenn die Arretierungsmittel derartig ausgestaltet sind, dass das erste und zweite Bauteil in einer Relativposition zueinander arretiert sind bzw. werden, wenn ein entsprechender Konnektor einer Leitung an den Anschlussport des zweiten Bauteils angekoppelt ist bzw. wird, und dass die Arretierung durch Abkoppeln des Konnektors vom Anschlussport aufhebbar ist, so dass bei abgekoppeltem Konnektor keine Arretierung vorliegt. Diese Variante kann vorteilhaft sein für den Fall, dass im Normalfall eine gewünschte Relativposition beibehalten werden soll, z.B. um ein Aufwickeln der Leitung zu verhindern.

Gemäss einer dritten Variante ist es bevorzugt, wenn die Arretierungsmittel derartig ausgestaltet sind, dass sie über ein oder mehrere Betätigungselemente aktivierbar und/oder deaktivierbar sind, wobei es gemäss einer Untervariante bevorzugt ist, wenn die Arretierungsmittel ohne Betätigung der Betätigungselemente arretiert sind. Hierdurch ergibt sich eine einfache Wiederauffindbarkeit des Anschlussports nach einem vorübergehenden Abkoppeln des Konnektors von der Anordnung und ein "Aufwickeln" der angekoppelten Leitung wird sicher verhindert. Gemäss einer besonders bevorzugten Untervariante können die Arretierungsmittel über die Betätigungselemente wahlweise aktiviert oder deaktiviert werden, so dass in jedem Zustand je nach Wunsch eine Verdrehung zwischen dem ersten und dem zweiten Bauteil zugelassen oder unterbunden werden kann.

Bei den mit Arretierungsmittel versehenen Ausführungsformen der Anordnung sind die Arretierungsmittel bevorzugterweise derartig ausgestaltet, dass die Arretierung formschlüssig an diskreten Positionen oder reibschlüssig an beliebigen Positionen erfolgen kann. Je nach Anwendung und konstruktiver Ausgestaltung kann die eine oder die andere Variante bevorzugter sein.

In noch einer weiteren bevorzugten Ausführungsform der Anordnung ist die Kanüle als flexible Kanüle ausgebildet, wobei es bevorzugt ist, wenn diese zur Ermöglichung eines Einstechens in das Gewebe eines Patienten in ihrem Kanülenkanal von einer nach der Applikation durch Einstechen entfernbaren Führungsnadel durchsetzt ist. Solche so genannten Softkanülen eignen sich besonders gut für den längeren Verbleib im subkutanen Gewebe eines Patienten, da sie praktisch keine Irritationen im Bereich der Einstichstelle hervorrufen.

In noch einer weiteren bevorzugten Ausführungsform der Anordnung trägt die Aussenseite des ersten Bauteils, auf welcher die Kanüle von diesem absteht, eine mit Vorteil von einem Pflaster gebildete Klebeschicht zur Befestigung des ersten Bauteils durch Kleben auf der Haut eines Patienten. Dabei ist die Klebeschicht von einer Schutzfolie bedeckt, welche erst kurz vor der Applikation der Anordnung entfernt wird. Durch eine solche Klebeschicht lässt sich die Anordnung beim Applizieren sicher und komfortabel auf der Haut des Patienten befestigen.

In noch einer weiteren bevorzugten Ausführung umfasst die Anordnung zusätzlich einen entsprechenden Konnektor zur Ankopplung einer Leitung zur Zuführung oder zur Abführung einer Flüssigkeit, wobei es bevorzugt ist, wenn die Anordnung einen entsprechenden Konnektor mit einer solchen Leitung umfasst.

Es wird auch die Verwendung der Anordnung nach einem der vorangehenden Ansprüche zum subkutanen Zuführen eines flüssigen Medikaments beschrieben, zum Beispiel zum subkutanen Zuführen von Insulin, in den Körper eines Patienten. Bei solchen Verwendungen treten die Vorteile der Erfindung besonders deutlich zu Tage.

### KURZE BESCHREIBUNG DER ZEICHNUNGEN

Weitere bevorzugte Ausführungen der Erfindung ergeben sich aus den abhängigen Ansprüchen und aus der nun folgenden Beschreibung anhand der Figuren. Dabei zeigen:
Fig. 1 eine perspektivische Draufsicht auf eine erste erfindungsgemässe Anordnung mit konnektierter Zuführungsleitung;
Fig. 2 eine perspektivische Draufsicht auf die Anordnung aus Fig. 1 bei entfernter Zuführungsleitung;
Fig. 3 einen Vertikalschnitt quer zur Längsrichtung der Zuführungsleitung durch einen Teil der in Fig. 1 dargestellten Anordnung;
Fig. 4 eine Darstellung wie Fig. 3 der Anordnung aus Fig. 2;
Fig. 5 eine Draufsicht auf die Anordnung aus Fig. 1;
Fig. 6 eine perspektivische Ansicht des den Anschlussport bereitstellenden zweiten Bauteils der Anordnung aus Fig. 1;
Fig. 7 eine perspektivische Draufsicht auf eine zweite erfindungsgemässe Anordnung im applizierten Zustand mit konnektierter Zuführungsleitung;
Fig. 8 eine perspektivische Draufsicht auf die Anordnung aus Fig. 7 bei entfernter Zuführungsleitung;
Fig. 9 einen Vertikalschnitt in Längsrichtung der Zuführungsleitung durch die in Fig. 7 dargestellte Anordnung;
Fig. 10 eine Darstellung wie Fig. 9 der Anordnung aus Fig. 8;
Fig. 11 einen Vertikalschnitt durch die in Fig. 7 dargestellte Anordnung angeordnet in einem Schutzgehäuse;
Fig. 12 einen Vertikalschnitt durch die in Fig. 7 dargestellte Anordnung direkt vor der Applikation;
Fig. 13 eine perspektivische Draufsicht auf eine dritte erfindungsgemässe Anordnung im applizierten Zustand mit konnektierter Zuführungsleitung;
Fig. 14 eine Seitenansicht der Anordnung aus Fig. 13 gesehen aus der Konnektierungsrichtung der Zuführungsleitung;
Fig. 15 einen Vertikalschnitt in Längsrichtung der Zuführungsleitung durch die in Fig. 13 dargestellte Anordnung;
Fig. 16 eine perspektivische Draufsicht auf eine vierte erfindungsgemässe Anordnung im applizierten Zustand mit konnektierter Zuführungsleitung;
Fig. 17 eine perspektivische Draufsicht auf die Anordnung aus Fig. 16 bei entfernter Zuführungsleitung;
Fig. 18 einen Vertikalschnitt in Längsrichtung der Zuführungsleitung durch die in Fig. 16 dargestellte Anordnung;
Fig. 19 eine perspektivische Draufsicht auf eine fünfte erfindungsgemässe Anordnung im applizierten Zustand mit konnektierter Zuführungsleitung;
Fig. 20 einen Vertikalschnitt in Längsrichtung der Zuführungsleitung durch die in Fig. 19 dargestellte Anordnung;
Fig. 21 eine perspektivische Draufsicht auf eine sechste erfindungsgemässe Anordnung im applizierten Zustand;
Fig. 22 eine Draufsicht auf die Anordnung aus Fig. 21;
Fig. 23 eine Seitenansicht der Anordnung aus Fig. 21;
Fig. 24 einen Vertikalschnitt quer zur Ankopplungsrichtung der Zuführungsleitung durch die in Fig. 21 dargestellte Anordnung; und
Fig. 25 eine perspektivische Ansicht des die Kanüle tragenden ersten Bauteils der Anordnung aus Fig. 21 zusammen mit einem Teil der Arretierungsmittel.

### WEGE ZUR AUSFÜHRUNG DER ERFINDUNG

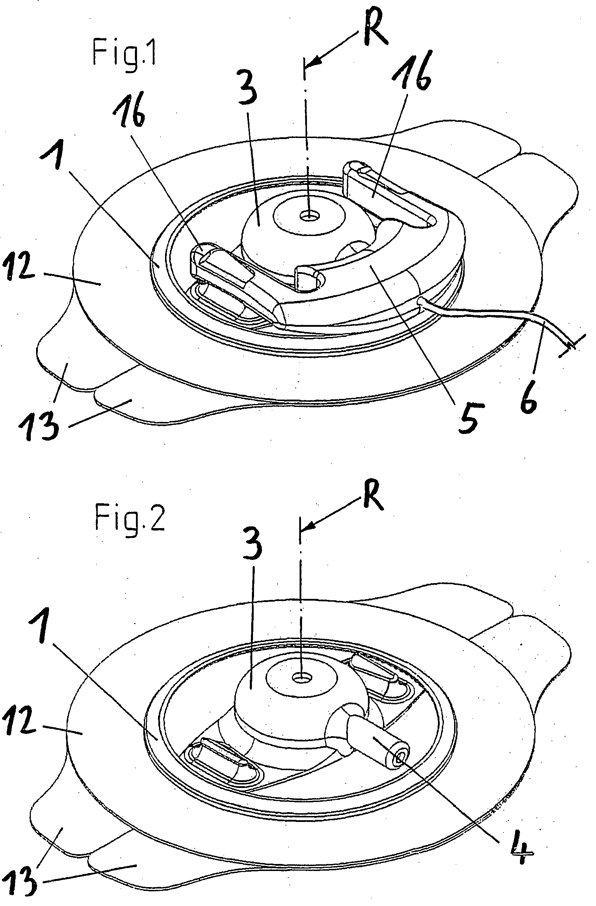

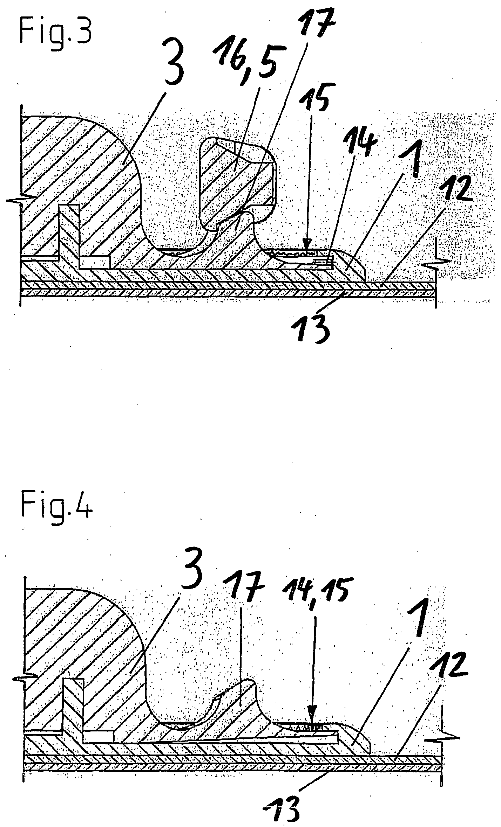

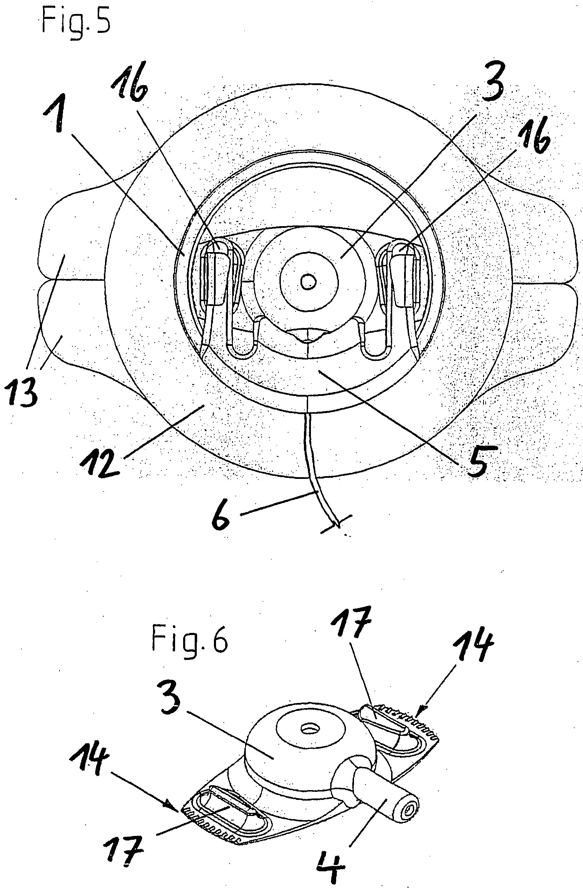

Eine erste bevorzugte Ausführungsform der erfindungsgemässen Anordnung in Form eines Infusionssets ist in den Figuren 1 und 2 in einer perspektivischen Draufsicht, in den Figuren 3 und 4 ausschnittweise im Längsschnitt und in Fig. 5 in der Draufsicht dargestellt, und zwar sowohl mit angekoppelter Zuführungsleitung für eine Infusionsflüssigkeit (Figuren 1, 3 und 5) als auch ohne Zuführungsleitung (Figuren 2 und 4). Wie in Zusammenschau dieser Figuren zu erkennen ist, umfasst das Infusionsset ein anspruchsgemässes erstes Bauteil 1, welches auf seiner Unterseite eine senkrecht abstehende Kanüle (nicht gezeigt) trägt. Die Unterseite des ersten Bauteils 1 wird von einem Pflaster 12 gebildet, dessen Klebefläche mit einem Pflasterschutzpapier 13 bedeckt ist. Ein anspruchsgemässes zweiten Bauteil 3 ist untrennbar und verdrehbar um eine vertikale Rotationsachse R herum, welche in der Längsachse der Kanüle verläuft, mit dem ersten Bauteil 1 verbunden. Das zweite Bauteil 3 bildet einen Anschlussport 4 für den Konnektor 5 der Zuführungsleitung 6 und wirkt derartig mit dem ersten Bauteil 1 zusammen, dass diese Bauteile 1, 3 in jeder rotatorischen Relativposition, welche sie zueinander einnehmen können, einen nach aussen abgedichteten Kanal (nicht gezeigt) zur Zuführung der Infusionsflüssigkeit vom Anschlussport 4 zur Kanüle bilden. Für den hier nicht gezeigten inneren Aufbau des Infusionssets, insbesondere bezüglich der Art und Weise, wie der Kanal zwischen der Kanüle und dem Anschlussport 4 durch das erste 1 und das zweite Bauteil 3 gebildet wird und wie die Kanüle mit dem ersten Bauteil 1 verbunden ist, wird auf die im Weiteren folgenden Ausführungsbeispiele verwiesen, in denen Konstruktionen im Detail aufgezeigt werden, welche auch für die hier vorliegende Ausführungsform geeignet sind.

Wie weiter in Zusammenschau der Figuren 3 und 4 mit Fig. 6, welche eine perspektivische Darstellung des zweiten Bauteils 3 zeigt, zu erkennen ist, weist das zweite Bauteil 3 an der Oberseite seiner äussersten radialen Peripherie nach oben gerichtete Zähne 14 auf, welche bei Abwesenheit eines entsprechenden Konnektors 5 zum Ankoppeln der Zuführungsleitung 6 an den Anschlussport 4 unter einer durch das zweite Bauteil 3 erzeugten Federkraft federnd in entsprechende, gegenüberliegende und 360° umlaufende Zähne 15 des ersten Bauteils 1 eingreifen und dadurch bei nicht-konnektierter Zuführungsleitung die Verdrehbarkeit der beiden Bauteile 1, 3 zueinander aufhebt (siehe Fig. 4). Wird die Zuführungsleitung 6 mit ihrem Konnektor 5 an den Anschlussport 4 des zweiten Bauteils 3 angekoppelt, indem die Haltearme 16 des Konnektors, z.B. durch Druckausübung mit dem Daumen und Zeigefinger, unter elastischer Deformation derselben aufeinander zu bewegt werden, der Konnektor 5 gleichzeitig in einer Richtung senkrecht zur Rotationsachse R der beiden Bauteile 1, 3 bzw. zur Längsachse der Kanüle auf den Anschlussport 4 aufgesteckt wird und die Haltearme 16 sodann wieder losgelassen werden, wodurch sie sich wieder voneinander weg bewegen und unter Federvorspannung an Anschlagnocken 17 am zweiten Bauteil 3 anschlagen, so wird das zweite Bauteil 3 durch die Federkraft der Haltearme 16 im Bereich seiner äusseren radialen Peripherie nach unten verwölbt, wodurch seine Zähne 14 ausser Eingriff mit den Zähnen 15 des ersten Bauteils 1 gebracht werden und die beiden Bauteile 1, 3 gegeneinander verdrehbar werden. Gleichzeitig verrasten die Haltearme 16 in Ankopplungsrichtung des Konnektors 5 gesehen hinter den Anschlagnocken 17, so dass ein unbeabsichtigtes Abziehen des Konnektors 5 vom Anschlussport 4 verunmöglicht wird. Wird der Konnektor 5 durch erneutes Zusammendrücken seiner Haltearme 16 und gleichzeitiges Bewegen desselben entgegen der Ankopplungsrichtung vom Infusionsset abgekoppelt, so wölbt sich das zweite Bauteil 3 im Bereich seiner äusseren radialen Peripherie wieder nach oben, bis seine Zähne 14 federnd in die Zähne 15 des ersten Bauteils 1 eingreifen und dadurch beide Bauteile 1, 3 in der gerade zueinander eingenommenen Relativposition arretiert werden.

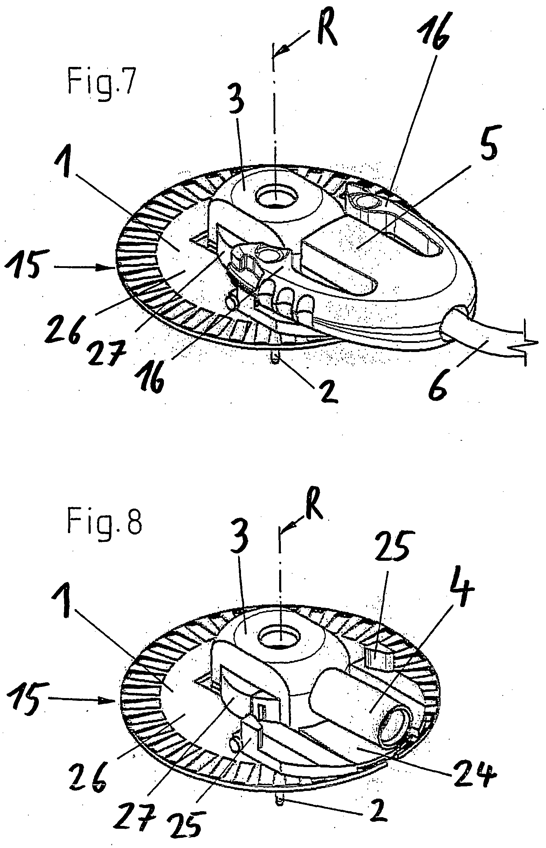

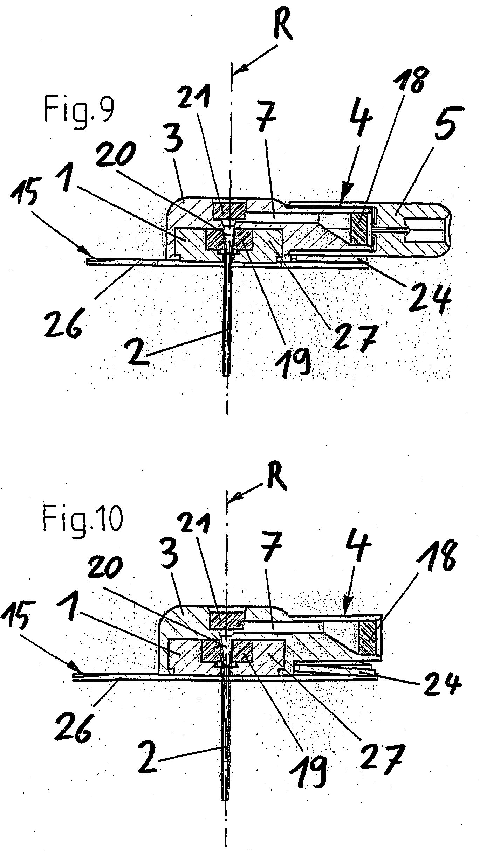

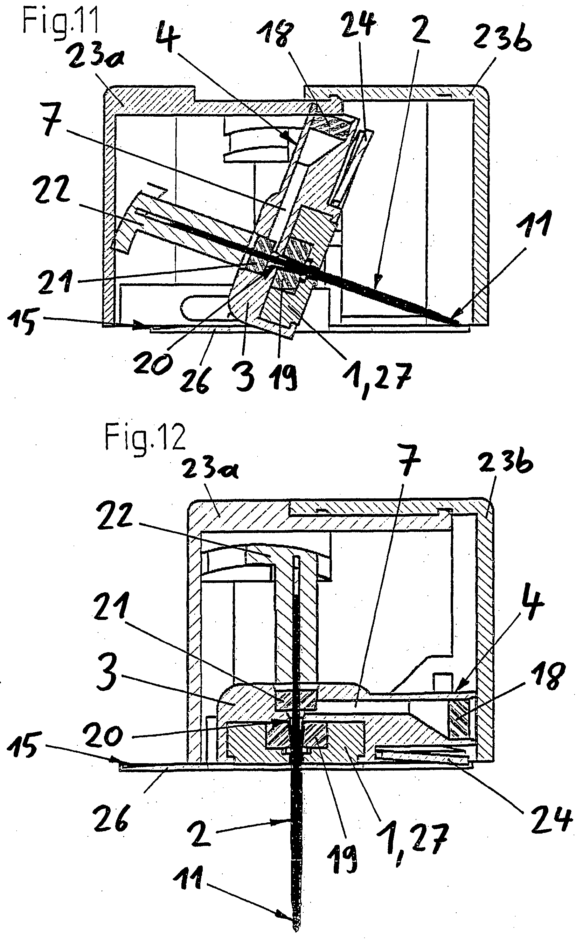

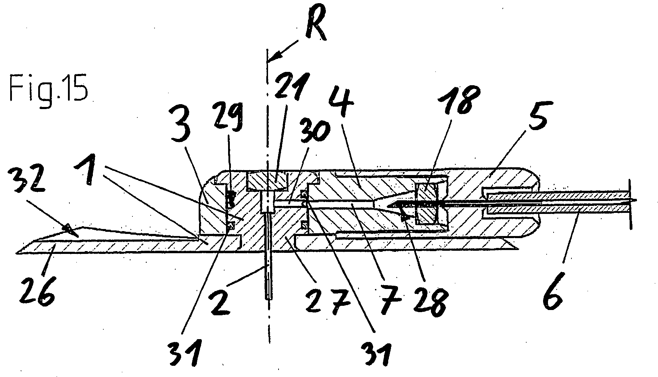

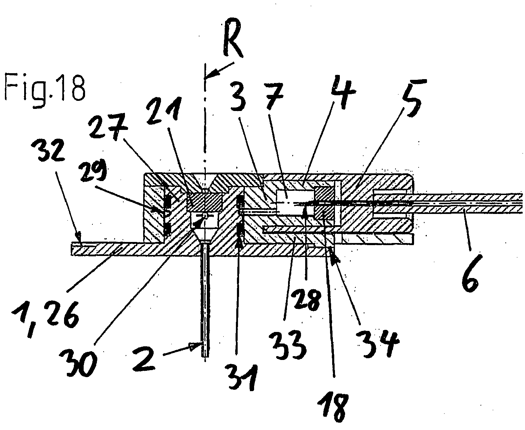

Die Figuren 7 bis 10 zeigen eine zweite Ausführungsform der erfindungsgemässen Anordnung in Form eines applizierten Infusionssets in perspektivischen Draufsichten und in Längsschnitten, und zwar einmal mit angekoppelter Zuführungsleitung (Figuren 7 und 9) und einmal ohne Zuführungsleitung (Figuren 8 und 10). Wie in Zusammenschau dieser Figuren zu erkennen ist, weist das Infusionsset wiederum ein anspruchsgemässes erstes Bauteil 1 auf, welches auf seiner Unterseite eine senkrecht abstehende Kanüle 2 aus einem flexiblen Material trägt. Auf der Oberseite des ersten Bauteils 1 ist ein anspruchsgemässes zweites Bauteil 3 angeordnet, welches einen Anschlussport 4 für die Ankopplung eines entsprechenden Konnektors 5 einer Zuführungsleitung 6 für Infusionsflüssigkeit bildet. Das erste 1 und das zweite Bauteil 3 sind untrennbar und um eine vertikale Rotationsachse R herum relativ zueinander verdrehbar miteinander verbunden, wobei die Rotationsachse R identisch mit der Längsachse der Kanüle 2 ist. Dabei bilden das erste 1 und das zweite Bauteil 3 in jeder rotatorischen Relativposition, die sie zueinander einnehmen können, einen flüssigkeitsdichten Kanal 7 zur Zuführung von Infusionsflüssigkeit vom Anschlussport 4 zur Kanüle 2. Wie insbesondere aus den Figuren 9 und 10 ersichtlich ist, ist der Kanal 7 anschlussportseitig von einem ersten Septum 18 begrenzt, welches der flüssigkeitsdichten Ankopplung der Zuführungsleitung 6 dient, indem es beim Ankoppeln des Konnektors 5 von einer in diesem angeordneten Zuführungskanüle (hier nicht gezeigt, jedoch wie in den Figuren 15 und 18 der folgenden Ausführungsbeispiele) durchstossen wird. Der flüssigkeitsdichte Übergang zwischen dem ersten 1 und dem zweiten Bauteil 3 ist mittels eines zweiten Septums 19 realisiert, welches von dem ersten Bauteil 1 bereitgestellt wird und von einem Kanülenfortsatz 20 des zweiten Bauteils 3, welcher sich in der Rotationsachse R der Bauteile 1, 3 erstreckt, durchsetzt ist. Wie in Zusammenschau mit den Figuren 11 und 12 erkennbar ist, welche Vertikalschnitte durch die in Fig. 7 dargestellte Anordnung vor der Applikation angeordnet in einem speziellen Schutzgehäuse 23a, 23b zeigen, ist der Kanal 7 direkt gegenüberliegend der Eintrittsöffnung der Kanüle 2 und in geradliniger Verlängerung des Kanülenkanals von einem weiteren Septum 21 begrenzt, welches einen Teil der Oberseite des zweiten Bauteils 3 bildet und im Ursprungszustand von einer Führungsnadel 11 durchsetzt ist. Diese Führungsnadel 11 stützt die Kanüle 2 beim Applizieren des Infusionssets durch Einstechen der Kanüle 2 in den Körper eines Patienten und wird nach dem Applizieren mit Hilfe eines an ihrem äusseren Ende angeordneten Rückziehelements 22 aus dem Kanülenkanal und dem weiteren Septum 21 herausgezogen und vom Infusionsset entfernt.

Das Schutzgehäuse 23a, 23b wird von zwei Gehäusehälften 23a, 23b gebildet, welche, ausgehend von der in Fig. 11 dargestellten Ursprungssituation, in welcher das Infusionsset in den Handel kommt und in welcher es geschützt innerhalb des Gehäuses 23a, 23b angeordnet ist, zusammenschiebbar sind. Wie zu erkennen ist, ist das erste Bauteil 1 bei dieser Ausführungsform von einem kreisrunden Tellerelement 26 und einem Zentralkörper 27, welcher die Kanüle 2 trägt und mit dem zweiten Bauteil 3 verbunden ist, gebildet, wobei das Tellerelement 26 und der Zentralkörper 27 in der Ursprungssituation gemäss Fig. 11 schwenkbar zueinander ausgebildet sind. Die beiden Gehäusehälften 23a, 23b weisen Führungen auf, über welche beim Zusammenschieben derselben der Zentralkörper 27 mit dem darauf angeordneten zweiten Bauteil 3 und der von der Führungsnadel 11 durchdrungenen Kanüle 2 verschwenkt wird, bis die von der Führungsnadel 11 durchdrungene Kanüle 2 unter einem rechten Winkel vom Gehäuse 23a, 23b und vom Tellerelement 26 absteht. In dieser Situation, welche in Fig. 12 dargestellt ist und die Situation direkt vor der Applikation des Infusionssets zeigt, verrasten sowohl die beiden Gehäusehälften 23a, 23b als auch das Tellerelement 26 und der Zentralkörper 27 unlösbar miteinander und das Gehäuse 23a, 23b bildet einen Applikationsgriff, welcher zum Applizieren des Infusionssets ergriffen wird und nach dem Applizieren entgegen der Einstichrichtung der Kanüle 2 vom Infusionsset entfernt wird, wobei gleichzeitig die Führungsnadel 11 mit dem Rückziehelement 22 aus der Kanüle 2 und dem weiteren Septum 21 herausgezogen und vom Infusionsset entfernt wird.

Wie weiter aus einer Zusammenschau der Figuren 7 bis 10 zu erkennen ist, weist das Tellerelement 26 des ersten Bauteils 1 an der Umfangsbegrenzung seiner Oberseite nach oben gerichtete Zähne 15 auf, in welche bei Abwesenheit eines entsprechenden Konnektors 5 zum Ankoppeln der Zuführungsleitung 6 an den Anschlussport 4 des zweiten Bauteils 3 ein an der Unterseite einer von dem zweiten Bauteil 3 gebildeten Verriegelungswippe 24 gebildeter Vorsprung eingreift und dadurch bei nicht-konnektierter Zuführungsleitung 6 die Verdrehbarkeit der beiden Bauteile 1, 3 zueinander aufhebt (siehe Figuren 8 und 10). Wird die Zuführungsleitung 6 mit ihrem Konnektor 5 an den Anschlussport 4 des zweiten Bauteils 3 angekoppelt, indem der Konnektor 5 in Richtung senkrecht zur Rotationsachse R der beiden Bauteile 1, 3 bzw. zur Längsachse der Kanüle 2 auf den Abschlussport 4 aufgesteckt wird, wobei dessen Haltearme 16 jeweils hinter einem zugeordneten Haltenocken 25 verrasten, so dass ein unbeabsichtigtes Abziehen des Konnektors 5 vom Anschlussport 4 verunmöglicht wird, so wird die Verriegelungswippe 24 durch den Konnektor 5 umgeschwenkt. Dadurch wird der an Ihrer Unterseite gebildete Vorsprung ausser Eingriff mit den Zähnen 15 des ersten Bauteils 1 gebracht und die beiden Bauteile 1, 3 werden gegeneinander verdrehbar. Wird der Konnektor 5 wieder entfernt, wozu die Haltearme 16 zum Aussereingriffbringen mit den Haltenocken 25 durch Druckausübung mit dem Daumen und Zeigefinger aufeinander zu bewegt werden müssen, während der Konnektor 5 entgegen der Ankopplungsrichtung vom Infusionsset entfernt wird, so kippt die Verriegelungswippe 24 federunterstützt wieder in ihre vorherige Ausgangsstellung zurück und arretiert die beiden Bauteile 1, 3 in der gerade zueinander eingenommenen rotatorischen Relativposition.

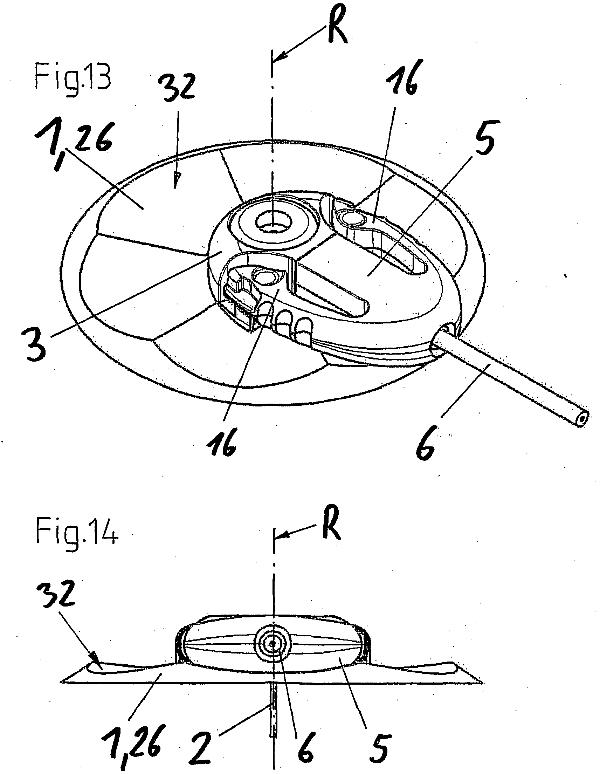

Die Figuren 13 bis 15 zeigen eine dritte Ausführungsform der erfindungsgemässen Anordnung in Form eines applizierten Infusionssets mit konnektierter Zuführungsleitung, einmal in perspektivischer Draufsicht (Fig. 13), einmal in der Seitenansicht aus der Konnektierungsrichtung der Zuführungsleitung gesehen (Fig. 14) und einmal im Vertikalschnitt in Längsrichtung der Zuführungsleitung (Fig. 15). Wie in Zusammenschau dieser Figuren zu erkennen ist, weist das Infusionsset ein anspruchsgemässes erstes Bauteil 1 auf, welches im wesentlichen von einem kreisrunden Tellerelement 26 und einem Zentralkörper 27 gebildet wird, welcher die Kanüle 2 trägt. Im oberen Bereich ist der Zentralkörper 27 von einem anspruchsgemässen zweiten Bauteil 3 umfangsmässig umschlossen, welches einen Anschlussport 4 für die Ankopplung eines entsprechenden Konnektors 5 einer Zuführungsleitung 6 für Infusionsflüssigkeit bildet. Das erste 1 und das zweite Bauteil 3 sind untrennbar und relativ zueinander verdrehbar um eine vertikale Rotationsachse R herum miteinander verbunden, wobei die Rotationsachse R identisch ist mit der Längsachse der Kanüle 2. Dabei bilden das erste 1 und das zweite Bauteil 3 in jeder rotatorischen Relativposition, die sie zueinander einnehmen können, einen flüssigkeitsdichten Kanal 7 zur Zuführung von Infusionsflüssigkeit vom Anschlussport 4 zur Kanüle 2. Wie aus Fig. 15 ersichtlich ist, ist der Kanal 7 anschlussportseitig von einem ersten Septum 18 begrenzt, welches der flüssigkeitsdichten Ankopplung der Zuführungsleitung 6 dient, indem es beim Ankoppeln des Konnektors 5 von einer in diesem angeordneten Zuführungskanüle 28 durchstossen wird. Der flüssigkeitsdichte Übergang zwischen dem ersten 1 und dem zweiten Bauteil 3 ist mittels zweier in Längsrichtung der Kanüle 2 voneinander beabstandeter O-Ringe 31 realisiert, welche vom Zentralkörper 27 getragen werden. Zwischen diesen O-Ringen 31 weist der Zentralkörper 27 eine Umfangsnut 29 auf, welche über eine radiale Bohrung 30 mit dem Kanüleneintritt in Verbindung steht. Wie weiter erkennbar ist, ist der Kanal 7 direkt gegenüberliegend der Eintrittsöffnung der Kanüle 2 und in geradliniger Verlängerung des Kanülenkanals von einem weiteren Septum 21 begrenzt, welches einen Teil der Oberseite des Zentralkörpers 27 des ersten Bauteils 1 bildet und im Ursprungszustand von einer Führungsnadel (hier nicht gezeigt) durchsetzt ist. Diese Führungsnadel stützt die Kanüle 2 beim Einstechen in den Körper eines Patienten und wird nach dem Applizieren aus dem Kanülenkanal und dem Septum 21 herausgezogen und vom Infusionsset entfernt.

Wie in Fig. 13 zu erkennen ist, weist das Tellerelement 26 des ersten Bauteils 1 an seiner Oberseite sechs identische, umfangsmässig in gleichmässiger Teilung angeordnete muldenartige Vertiefungen 32 auf, deren Oberflächenkontur einen in radialer Richtung gesehen konstanten Profilquerschnitt aufweist. Wie aus Fig. 14 zu ersehen ist, weist der zugeordnete Konnektor 5 der Zuführungsleitung 6 an seiner Unterseite eine entsprechende Gegenkontur auf und ist derartig auf dem Anschlussport 4 des zweiten Bauteils 3 geführt, dass er nur bei Relativpositionen zwischen dem ersten 1 und dem zweiten Bauteil 3, bei denen sich der Anschlussport 4 mittig über einer der Vertiefungen 32 befindet, auf den Anschlussport 4 aufgesteckt werden kann und dann die Bauteile 1, 3 in der gerade eingenommenen Relativposition arretiert, indem er mit der Gegenkontur seiner Unterseite in die jeweilige Vertiefung 32 eingreift. Bei dieser Ausführungsform lassen sich das erste 1 und das zweite Bauteil 3 in sechs verschiedenen rotatorischen Relativpositionen zueinander arretieren, wobei das Ankoppeln des Konnektors 5 an den Anschlussport 4 nur in einer dieser Relativpositionen möglich ist und dann zwangsläufig eine Arretierung der Bauteile 1, 3 in der Relativposition zur Folge hat. Auch hier weist der Konnektor 5 Haltearme 16 auf, mit welchen er beim Aufstecken auf den Anschlussport 4 hinter Haltenocken (nicht gezeigt) einrastet, so dass ein unbeabsichtigtes Abziehen des Konnektors 5 vom Anschlussport 4 verhindert wird, und welche zum Abkoppeln des Konnektors 5 vom Infusionsset unter Druckausübung mit dem Daumen und Zeigefinger aufeinander zu bewegt werden müssen, damit der Konnektor 5 entgegen der Ankopplungsrichtung entfernt werden kann. Wird der Konnektor 5 wie zuvor beschrieben wieder vom Infusionsset entfernt, so sind die beiden Bauteile 1, 3 wieder zueinander verdrehbar.

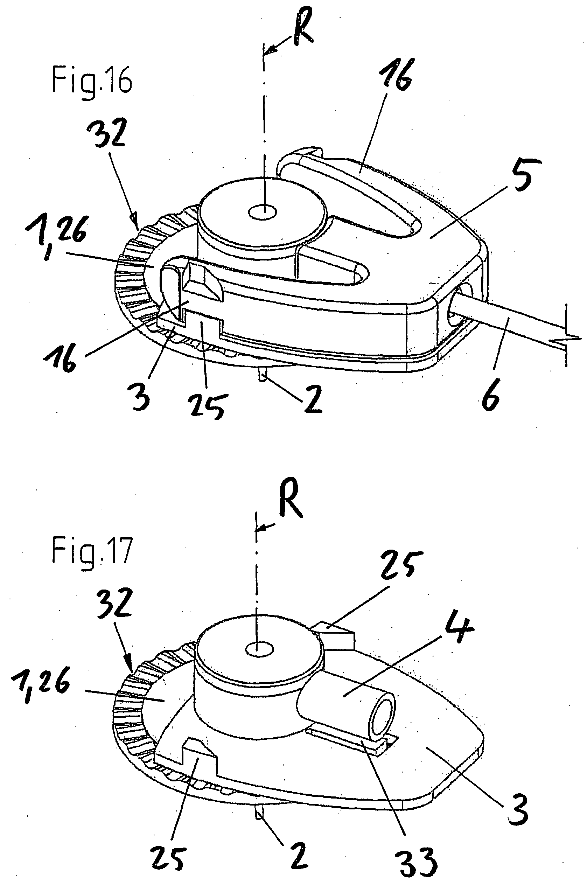

Die Figuren 16 bis 18 zeigen eine vierte Ausführungsform der erfindungsgemässen Anordnung in Form eines applizierten Infusionssets, einmal in perspektivischer Draufsicht mit konnektierter Zuführungsleitung (Fig. 16), einmal in perspektivischer Draufsicht ohne Zuführungsleitung (Fig. 17) und einmal im Vertikalschnitt in Längsrichtung der konnektierten Zuführungsleitung (Fig. 18). Das hier gezeigte Infusionsset weist einen ähnlichen Aufbau wie das zuvor beschriebene auf, jedoch mit dem Unterschied, dass hier das Tellerelement 26.einstückig mit dem Zentralkörper 27 ausgebildet ist und an seiner Oberseite an Stelle der sechs grossen Vertiefungen entlang seiner Umfangsbegrenzung eine Vielzahl identischer kleiner, umfangsmässig in gleichmässiger Teilung angeordneter Vertiefungen 32 aufweist, so dass im Bereich des Randes eine gewellte Oberflächenkontur entsteht. Ebenfalls im Gegensatz zu der vorherigen Ausführungsform weist das zweite Bauteil 3 hier einen federelastischen Arretierungsarm 33 auf, welcher bei nicht-konnektierter Zuführungsleitung 6 mit einem an seiner Unterseite gebildeten Rastvorprung 34 auf der gewellten Oberflächenkontur des Tellerelements 26 aufliegt und so zusammen mit dieser einen Ratschenmechanismus bildet, der bewirkt, dass zum Verdrehen des zweiten Bauteils 3 gegenüber dem ersten Bauteil 1 eine bestimmte Kraft aufgewendet werden muss und dass diese Bauteile 1, 3 jeweils diskrete Relativpositionen zueinander einnehmen. Wird nun ein entsprechender Konnektor 5 auf den Anschlussport 4 des zweiten Bauteils 3 aufgesteckt, wie dies in den Figuren 16 und 18 gezeigt ist, so wird der Arretierungsarm 33 durch den Konnektor 5 am Ausweichen nach oben gehindert und das erste 1 und das zweite Bauteil 3 werden in der gerade eingenommenen Relativposition arretiert. Bei dieser Ausführungsform lassen sich das erste 1 und das zweite Bauteil 3 also in einer Vielzahl diskreter Relativpositionen zueinander arretieren, wobei das Ankoppeln des Konnektors 5 an den Anschlussport 4 nur in einer dieser Relativpositionen möglich ist und dann zwangsläufig eine Arretierung der Bauteile 1, 3 in der Relativposition zur Folge hat. Bei diskonnektiertem Konnektor 5 sind das erste 1 und das zweite Bauteil 3 entgegen einer vom zuvor beschriebenen Ratschenmechanismus definierten Kraft gegeneinander verdrehbar, wobei sie jedoch nach Aufhebung einer Verdrehkraft diskrete Positionen zueinander einnehmen. Auch hier weist der Konnektor 5 Haltearme 16 auf, mit welchen er beim Aufstecken auf den Anschlussport 4 hinter Haltenocken 25 einrastet, so dass ein unbeabsichtigtes Abziehen des Konnektors 5 vom Anschlussport 4 verunmöglicht wird, und welche zum Abkoppeln des Konnektors 5 vom Infusionsset aufeinander zu bewegt werden müssen, damit der Konnektor 5 entgegen der Ankopplungsrichtung entfernt werden kann. Der übrige Aufbau dieser Ausführungsform, insbesondere bezüglich der konstruktiven Ausgestaltung der Abdichtungen 18, 21, 31 und des Kanals 7, entspricht im wesentlichen dem vorangehenden Ausführungsbeispiel, weshalb hier nicht nochmals im Detail darauf eingegangen wird. Hier nicht explizit beschriebene aber mit Bezugs-, ziffern versehene Elemente haben die gleiche Funktion wie die Bauteile mit entsprechender Bezugsziffer bei den vorher beschriebenen Ausführungen.

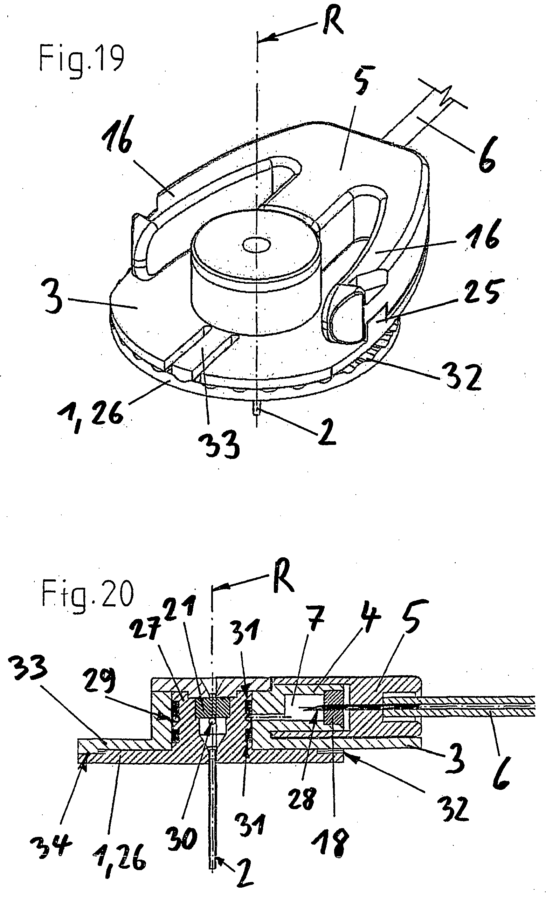

Die Figuren 19 und 20 zeigen eine fünfte Ausführungsform der erfindungsgemässen Anordnung in Form eines applizierten Infusionssets, einmal in perspektivischer Draufsicht mit konnektierter Zuführungsleitung (Fig. 19) und einmal im Vertikalschnitt in Längsrichtung der konnektierten Zuführungsleitung (Fig. 20). Das hier gezeigte Infusionsset ist identisch zu dem zuvor beschriebenen bis auf den Unterschied, dass hier der Arretierungsarm 33 an einer dem Anschlussport gegenüberliegenden Umfangsposition angeordnet ist und dadurch auch bei konnektierter Zuführungsleitung 6 zurückfedern kann. Entsprechend ist bei dieser Ausführungsform das zweite Bauteil 3 sowohl bei angekoppeltem Konnektor 5 als auch bei entferntem Konnektor 5 immer entgegen der durch den Ratschenmechanismus definierten Kraft gegenüber dem ersten Bauteil 1 verdrehbar, wobei diese Bauteile 1, 3 dann jeweils diskrete Relativpositionen zueinander einnehmen. Hier nicht explizit beschriebene aber mit Bezugsziffern versehene Elemente haben die gleiche Funktion wie die Bauteile mit entsprechender Bezugsziffer bei der zuvor beschriebenen Ausführungsform.

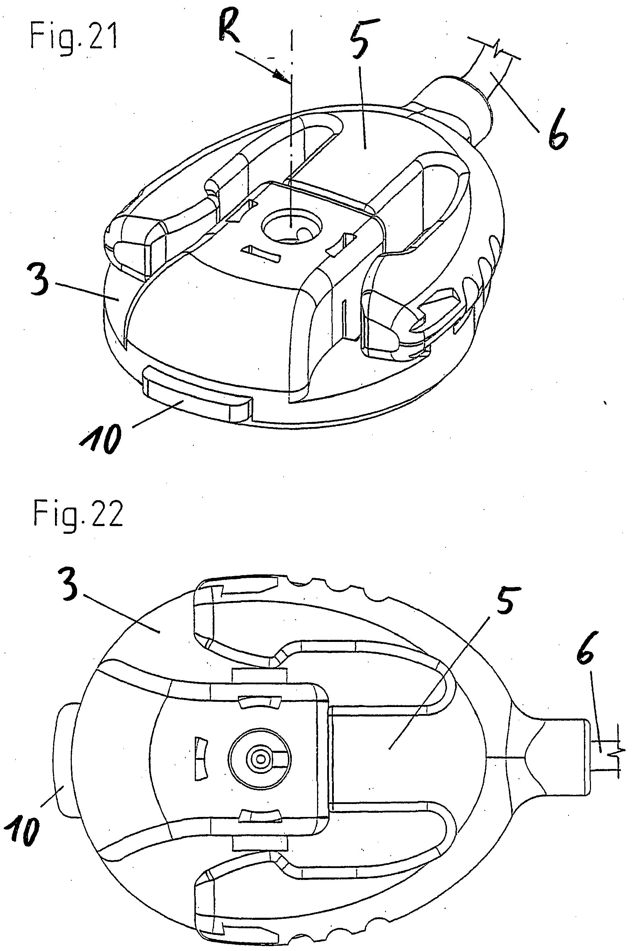

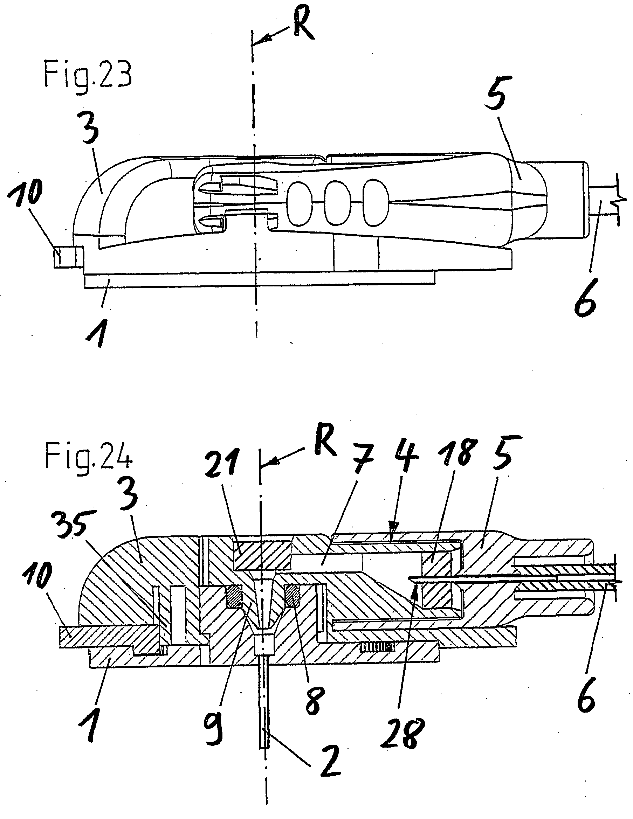

Die Figuren 21 bis 24 zeigen eine sechste Ausführungsform der erfindungsgemässen Anordnung in Form eines applizierten Infusionssets mit konnektierter Zuführungsleitung, einmal in perspektivischer Draufsicht (Fig. 21), einmal in der Draufsicht (Fig. 22), einmal in der Seitenansicht (Fig. 23) und einmal im Vertikalschnitt in Längsrichtung der konnektierten Zuführungsleitung (Fig. 24). Der innere Aufbau dieser Ausführungsform bezüglich der konstruktiven Ausgestaltung der Abdichtungen 8, 18 und 21 des Kanals 7 entspricht im wesentlichen dem anhand der Figuren 7 bis 10 diskutierten zweiten Ausführungsbeispiel. Wie in Zusammenschau der Figuren 21 bis 24 zu erkennen ist, weist das Infusionsset wiederum ein anspruchsgemässes erstes Bauteil 1 auf, welches auf seiner Unterseite eine senkrecht abstehende Kanüle 2 aus einem flexiblen Material trägt. Auf der Oberseite des ersten Bauteils 1 ist ein anspruchsgemässes zweites Bauteil 3 angeordnet, welches einen Anschlussport 4 für die Ankopplung eines entsprechenden Konnektors 5 einer Zuführungsleitung 6 für Infusionsflüssigkeit bildet. Das erste 1 und das zweite Bauteil 3 sind untrennbar und relativ zueinander verdrehbar um eine vertikale Rotationsachse R herum miteinander verbunden, wobei die Rotationsachse R identisch ist mit der Längsachse der Kanüle 2. Dabei bilden das erste 1 und das zweite Bauteil 3 in jeder rotatorischen Relativposition, die sie zueinander einnehmen können, einen flüssigkeitsdichten Kanal 7 zur Zuführung von Infusionsflüssigkeit vom Anschlussport 4 zur Kanüle 2. Auch hier ist der Kanal 7 anschlussportseitig von einem ersten Septum 18 begrenzt, welches der flüssigkeitsdichten Ankopplung der Zuführungsleitung 6 dient, indem es beim Ankoppeln des Konnektors 5 von einer in diesem angeordneten Zuführungskanüle 28 durchstossen wird. Der flüssigkeitsdichte Übergang zwischen dem ersten 1 und dem zweiten Bauteil 3 ist hier mittels einer unter Druckspannung stehenden Gummidichtung 8 realisiert, welche von einem Anschlusszapfen 9 des zweiten Bauteils 3 durchsetzt ist, der sich in der Rotationsachse R der Bauteile 1, 3 erstreckt und in seinem Zentrum eine Bohrung aufweist, die einen Teil des Kanals 7 bildet. Direkt gegenüberliegend der Eintrittsöffnung der Bohrung des Zapfens 9 und in geradliniger Verlängerung des Kanülenkanals wird der Kanal 7 von einem weiteren Septum 21 begrenzt, welches einen Teil der Oberseite des zweiten Bauteils 3 bildet und im Ursprungszustand von einer Führungsnadel (nicht gezeigt) durchsetzt ist. Diese Führungsnadel stützt die Kanüle 2 beim Applizieren des Infusionssets durch Einstechen der Kanüle 2 in den Körper eines Patienten und wird nach dem Applizieren mit Hilfe eines an ihrem äusseren Ende angeordneten Rückziehelements aus dem Kanülenkanal und dem weiteren Septum 21 herausgezogen und vom Infusionsset entfernt.

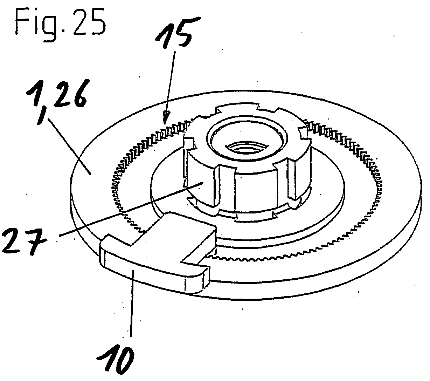

Wie insbesondere in Kombination mit Fig. 25 ersichtlich ist, welche eine perspektivische Ansicht des ersten Bauteils 1 zusammen mit einem Betätigungselement 10 einer noch genauer zu beschreibenden Arretierungsvorrichtung zeigt, ist das erste Bauteil 1 hier aus einem Tellerlement 26, welches eine umlaufende, radial nach innen gerichtete Innenverzahnung 15 aufweist, und einem Zentralkörper 27 gebildet, welcher im Zentrum des Tellerelements 26 nach oben von diesem absteht. Angeordnet zwischen dem ersten 1 und dem zweiten Bauteil 3 befindet sich an einer Umfangsposition, welche dem Anschlussport 4 gegenüberliegt, das auch in Fig. 25 gezeigte Betätigungselement 10, welches im zweiten Bauteil 3 radial geführt ist und mit einer vom zweiten Bauteil 3 gebildeten Federzunge 35 unter Federvorspannung radial nach aussen gedrückt wird, wo es mit einer von diesem gebildeten entsprechenden Gegenverzahnung (nicht gezeigt) in die Innenverzahnung 15 des ersten Bauteils 1 eingreift und dadurch ein Verdrehen der beiden Bauteile 1, 3 in der dargestellten Situation verunmöglicht. Wird in radialer Richtung nach innen eine Druckkraft auf das Betätigungselement 10 ausgeübt, so verschiebt sich dieses entgegen der Federkraft der Federzunge 35 radial nach innen, wodurch die Gegenverzahnung des Betätigungselements 10 ausser Eingriff mit der Innenverzahnung 15 gebracht wird und ein Verdrehen der zweiten Bauteils 3 gegenüber dem ersten Bauteil 1 möglich wird, unabhängig davon, ob ein Konnektor 5 an den Anschlussport 4 angekoppelt ist oder nicht. Wird das Betätigungselement 10 entlastet, so wird es durch die Federzunge 35 wieder radial nach aussen bewegt, bis die Gegenverzahnung des Betätigungselements 10 in die Innenverzahnung 15 des ersten Bauteils 1 eingreift und die beiden Bauteile 1, 3 in der gerade zueinander eingenommenen rotatorischen Relativposition arretiert. Auch hier erfolgt die Arretierung an diskreten Positionen.

## Patentansprüche

1. Anordnung zum Zuführen einer Flüssigkeit in den Körper eines Patienten oder zum Anführen einer Flüssigkeit aus dem Körper eines Patienten, umfassend
- ein erstes Bauteil (1) mit einem von diesem abstehenden Kanüle (2) zur Anordnung im Körper des Patienten;
- ein zweites Bauteil (3) mit einem Anschlussport (4) zum lösbaren Ankoppeln eines entsprechenden Konnektors (5) einer Leitung zum Zuführen oder abführen einer Flüssigkeit;
wobei das erste Bauteil (1) und das zweite Bauteil (3) derartig ausgestaltet und miteinander verbunden sind, dass sie untrennbar und relativ zueinander um eine parallel zur Längsachse der Kanüle verlaufende Rotationsachse (R) herum verdrehbar sind und in jeder Relativposition einen Kanal (7) zur Zuführung einer Flüssigkeit vom Anschlussport (4) zur Kanüle (2) oder zum Abführen einer Flüssigkeit von der Kanüle (2) zum Anschlussport (4) bilden, und wobei der Anschlussport (4) des zweiten Bauteils (3) derartig ausgebildet ist, dass das Ankoppeln und Abkoppeln eines entsprechenden Konnektors (5) jeweils in einer Richtung quer zur Längsachse der Kanüle (2) erfolgt,
**dadurch gekennzeichnet, dass**
Arretierungsmittel vorhanden sind zur Aufhebung der Verdrehbarkeit des ersten (1) und zweiten Bauteils (3) relativ zueinander durch Arretieren derselben in einer bestimmten Relativposition zueinander.

2. Anordnung nach Anspruch 1, **dadurch gekennzeichnet, dass** der Anschlussport (4) des zweiten Bauteils (3) derartig ausgebildet ist, dass das Ankoppeln und Abkoppeln eines entsprechenden Konnektors (5) jeweils senkrecht zur Längsachse der Kanüle (2) erfolgt.

3. Anordnung nach einem der vorangehenden Ansprüche, **dadurch gekennzeichnet, dass** die Kanüle (2) senkrecht oder nicht-senkrecht von einer im wesentlichen ebenen Aussenseite des ersten Bauteils (1) absteht und das erste (1) und das zweite Bauteil (3) um eine senkrecht zu dieser Aussenseite stehende Rotationsachse (R) herum relativ zueinander verdrehbar sind.

4. Anordnung nach einem der vorangehenden Ansprüche, **dadurch gekennzeichnet, dass** das erste (1) und das zweite Bauteil (3) um 360° oder um weniger als 360° zueinander verdrehbar sind.

5. Anordnung nach einem der vorangehenden Ansprüche, **dadurch gekennzeichnet, dass** die Verbindung zwischen dem ersten (1) und dem zweiten Bauteil (3) derartig ausgebildet ist, dass zum Gegeneinanderverdrehen der beiden Bauteile (1, 3) ein bestimmtes Drehmoment überwunden werden muss, insbesondere ein Drehmoment im Bereich zwischen 0,01 und 0,05 Nm, insbesondere zwischen 0,01 und 0,1 Nm.

6. Anordnung nach Anspruchz 5, **dadurch gekennzeichnet, dass** zur Erzeugung des zu überwindenden Drehmoments eine durch ein vorgespanntes elastisches Bauteil (8, 31) Reibung zwischen dem ersten (1) und dem zweiten Bauteil (3) erzeugende Anordnung vorhanden ist, insbesondere eine vorgespannte elastische Abdichtung (8, 31) und/oder ein Ratschenmechanismus.

7. Anordnung nach einem der vorangehenden Ansprüche, **dadurch gekennzeichnet, dass** die Arretierungsmittel derartig ausgestaltet sind, dass das erste (1) und zweite Bauteil (3) in einer Relativposition zueinander arretiert sind, wenn kein entsprechender Konnektor (5) einer Leitung (6) an den Anschlussport (4) des zweiten Bauteils (3) angekoppelt ist, und dass die Arretierung durch Ankoppeln eines entsprechenden Konnektors (5) an den Anschlussport (4) aufhebbar ist.

8. Anordnung nach einem der Ansprüche 1 bis 6, **dadurch gekennzeichnet, dass** die Arretierungsmittel derartig ausgestaltet sind, dass das erste (1) und zweite Bauteil (3) in einer Relativposition zueinander arretiert sind, wenn ein entsprechender Konnektor (5) einer Leitung (6) an den Anschluasport (4) des zweiten Bauteils (3) angekoppelt ist, und dass die Arretierung durch Abkoppeln des Konnektors (5) vom Anschlussport (4) aufhebbar ist.

9. Anordnung nach einem der Ansprüche 1 bis 6, **dadurch gekennzeichnet, dass** die Arretierungsmittel derartig ausgestaltet sind, dass sie über ein oder mehrere Betätigungselemente (10) aktivierbar und/oder deaktivierbar sind.

10. Anordnung nach einem der vorangehenden Ansprüche **dadurch gekennzeichnet, dass** die Arretierungsmittel derartig ausgestaltet sind, dass die Arretierung formschlüssig an diskreten Positionen oder reibschlüssig an beliebigen Positionen erfolgen kann.

11. Anordnung nach einem der vorangehenden Ansprüche, **dadurch gekennzeichnet, dass** die Kanüle (2) eine flexible Kanüle (2) ist, und insbesondere, dass diese in ihrem Kanülenkanal von einer entfernbaren Führungsnadel (11) durchsetzt ist, zur Ermöglichung eines Einstachens der Kanüle (2) in das Gewebe eines Patienten.

12. Anordnung nach einem der vorangehenden Ansprüche, dass die Aussenseite des ersten Bauteils (1), auf welcher die Kanüle (2) von diesem absteht, eine insbesondere von einem Pflaster (12) gebildete Klebeschicht trägt, zur Befestigung des ersten Bauteils (1) durch Kleben auf der Haut eines Patienten.

13. Anordnung nach einem der vorangehenden Ansprüche, des Weiteren umfassend einen entsprechenden Konnektor (5) zum Ankoppeln einer Leitung (6) zum Zuführen oder Abführen einer Flüssigkeit.

## Claims

1. An arrangement for introducing a liquid into a patient's body or for removing a liquid from a patient's body, comprising
- a first component (1) having a cannula (2) protruding from it for positioning in the body of the patient;
- a second component (3) with a connection port (4) for releasably coupling a corresponding connector (5) of a line for introducing or withdrawing a liquid;
wherein the first component (1) and the second component (3) are so designed and connected together that they are inseparable and rotatable relative to each other around an axis of rotation (R) running parallel to the longitudinal axis of the cannula and in each relative position form a channel (7) for feeding a liquid from the connection port (4) to the cannula (2) or for withdrawing a liquid from the cannula (2) to the connection port (4), and wherein the connection port (4) of the second component (3) is so designed that the coupling and uncoupling of a corresponding connector (5) take place in each case in a direction transverse to the longitudinal axis of the cannula (2),
**characterized in that**
locking means are present for cancelling the rotatability of the first (1) and second component (3) relative to one another by locking these in a particular relative position.

2. An arrangement according to claim 1, **characterized in that** the connection port (4) of the second component (3) is designed such that the coupling and uncoupling of a corresponding connector (5) take place in each case perpendicularly to the longitudinal axis of the cannula (2).

3. An arrangement according to any one of the preceding claims, **characterized in that** the cannula (2) projects out perpendicularly or non-perpendicularly from an essentially planar outer side of the first component (1) and the first (1) and second component (3) are rotatable relative to one another around an axis of rotation (R) perpendicular to this outer side.

4. An arrangement according to any one of the preceding claims, **characterized in that** the first (1) and the second component (3) are rotatable with respect to each other by 360° or by less than 360°.

5. An arrangement according to any one of the preceding claims, **characterized in that** the connection between the first (1) and second component (3) is designed such that a certain torque must be exceeded to rotate the two components (1, 3) against each other, in particular a torque ranging between 0.01 and 0.05 Nm, in particular between 0.01 and 0.1 Nm.

6. An arrangement according to claim 5, **characterized in that** an arrangement is provided for generating friction between the first (1) and second component (3) by means of a prestressed elastic component (8, 31), in particular a prestressed elastic seal (8, 31) and/or a ratchet mechanism, in order to produce the torque to be exceeded.

7. An arrangement according to any one of the preceding claims, **characterized in that** the locking means is designed such that the first (1) and second component (3) are locked in a position relative to one another, when no corresponding connector (5) of a supply line (6) is coupled to the connection port (4) of the second component (3) and that the locking is released by coupling a corresponding connector (5) to the connection port (4).

8. An arrangement according to any one of claims 1 to 6, **characterized in that** the locking means is designed such that the first (1) and second component (3) are locked in a position relative to one another, when a corresponding connector (5) of a supply line (6) is coupled to the connection port (4) of the second component (3), and that the locking is releasable by uncoupling the connector (5) from the connection port (4).

9. An arrangement according to any one of claims 1 to 6, **characterized in that** the locking means is designed such that it can be activated and/or deactivated via one or more actuators.

10. An arrangement according to any one of the preceding claims, **characterized in that** the locking means is designed such that the locking can take place positively at discrete positions or by frictional resistance at any position.

11. An arrangement according to any one of the preceding claims, **characterized in that** the cannula (2) is a flexible cannula (2) and in particular this is passed through in a cannula channel thereof by a removable guide needle (11) to enable piercing into a patient's tissue.

12. An arrangement according to any one of the preceding claims, **characterized in that** the outer side of the first component (1) out from which the cannula (2) protrudes, carries an adhesive layer in particular composed of a medical plaster (12) for fastening the component (1) by bonding to the patient's skin.

13. An arrangement according to any one of the preceding claims, additionally including a corresponding connector (5) for coupling of a line (6) for feeding or discharging a fluid.

## Revendications

1. Dispositif destiné à délivrer un liquide dans le corps d'un patient ou à guider un liquide hors du corps d'un patient, comprenant
- une première pièce (1) avec une canule (2) faisant saillie à partir de celle-ci, destinée à être placée dans le corps du patient ;
- une deuxième pièce (3) avec port de raccordement (4) permettant d'accoupler de façon amovible un connecteur correspondant (5) d'une conduite destinée à délivrer ou à évacuer un liquide ;
dans lequel la première pièce (1) et la deuxième pièce (3) sont conçues et reliée l'une à l'autre de façon à pouvoir être tournée de manière inséparable et l'une par rapport à l'autre autour d'un axe de rotation (R) s'étendant parallèlement à l'axe longitudinal de la canule, et à former un canal (7) dans chaque position relative, pour l'alimentation d'un liquide du port de raccordement (4) vers la canule (2) ou pour l'évacuation d'un liquide de la canule (2) vers le port de raccordement (4), et dans lequel le port de raccordement (4) de la deuxième pièce (3) est conçu de façon à ce que l'accouplement ou le désaccouplement d'un connecteur correspondant (5) se fasse respectivement dans une direction transversale à l'axe longitudinal de la canule (2),
**caractérisé en ce que**
il est prévu des moyens de blocage destinés à empêcher la rotation de la première (1) et de la deuxième pièce (3) l'une par rapport à l'autre en bloquant celles-ci dans une première position relative l'une par rapport à l'autre.

2. Dispositif selon la revendication 1, **caractérisé en ce que** le port de raccordement (4) de la deuxième pièce (3) est conçu de telle façon que l'accouplement et le désaccouplement d'un connecteur correspondant (5) se fait respectivement perpendiculairement à l'axe longitudinal de la canule (2).

3. Dispositif selon l'une des revendications précédentes, **caractérisé en ce que** la canule (2) fait saillie perpendiculairement ou non-perpendiculairement à partir du côté extérieur plat de la première pièce (1), et la première (1) et la deuxième pièce (3) peuvent tourner l'une par rapport à l'autre autour d'un axe de rotation (R) s'étendant perpendiculairement à ce côté extérieur.

4. Dispositif selon l'une des revendications précédentes, **caractérisé en ce que** la première (1) et la deuxième pièce (3) peuvent tourner l'une par rapport à l'autre de 360° ou de moins de 360°.

5. Dispositif selon l'une des revendications précédentes, **caractérisé en ce que** la liaison entre la première (1) et la deuxième pièce (3) est conçue de telle façon qu'un certain moment de rotation doit être surmonté pour faire tourner les deux pièces (1, 3) l'une rapport à l'autre, en particulier un moment de rotation compris entre 0,01 et 0,05 Nm, en particulier entre 0,01 et 0,1 Nm.

6. Dispositif selon la revendication 5, **caractérisé en ce que** pour générer le moment de rotation à surmonter, il est prévu un dispositif générant un frottement entre la première (1) et la deuxième pièce (3) par le biais d'une pièce élastique précontrainte (8, 31), en particulier d'un moyen d'étanchéité élastique précontraint (8, 31) et/ou d'un mécanisme à rochet.

7. Dispositif selon l'une des revendications précédentes, **caractérisé en ce que** les moyens de blocage sont conçus de telle façon que la première (1) et la deuxième pièce (3) sont bloquées dans une position relative l'une par rapport à l'autre, lorsqu'aucun connecteur correspondant (5) d'une conduite (6) n'est accouplé au port de raccordement (4) de la deuxième pièce (3), et **en ce que** le blocage par accouplement d'un connecteur correspondant (5) au port de raccordement (4) peut être supprimé.

8. Dispositif selon l'une des revendications 1 à 6, **caractérisé en ce que** les moyens de blocage sont conçus de telle façon, que la première (1) et la deuxième pièce (3) sont bloquées dans une position relative l'une par rapport à l'autre, lorsqu'un connecteur correspondant (5) d'une conduite (6) est accouplé au port de raccordement (4) de la deuxième pièce (3), et **en ce que** le blocage peut être supprimé en désaccouplant le connecteur (5) du port de raccordement (4).

9. Dispositif selon l'une des revendications 1 à 6, **caractérisé en ce que** les moyens de blocage sont conçus de façon à pouvoir être activés et/ou désactivés par le biais d'un ou de plusieurs éléments d'actionnement (10).

10. Dispositif selon l'une des revendications précédentes, **caractérisé en ce que** les moyens de blocage sont conçus de manière à ce que le blocage peut se faire par complémentarité de forme en des positions discrètes ou par frottement en des positions au choix.

11. Dispositif selon l'une des revendications précédentes, **caractérisé en ce que** la canule (2) est une canule souple (2), et en particulier **en ce que** celle-ci est traversée par une aiguille de guidage amovible (11) dans son canal de canule, permettant de piquer la canule (1) dans le tissu d'un patient.

12. Dispositif selon l'une des revendications précédentes, **caractérisé en ce que** le côté extérieur de la première pièce (1), sur lequel la canule (2) fait saillie, porte une couche adhésive formée en particulier par un pansement (12), pour fixer la première pièce (1) par collage sur la peau d'un patient.

13. Dispositif selon l'une des revendications précédentes, comprenant en outre un connecteur correspondant (5) permettant d'accoupler une conduite (6) pour alimenter ou évacuer un liquide.
